## Europäisches Patentamt
## European Patent Office
## Office européen des brevets

(19)

(11) Veröffentlichungsnummer: **0 175 973 B1**

(12)

# EUROPÄISCHE PATENTSCHRIFT

(45) Veröffentlichungstag der Patentschrift:
**08.11.89**

(51) Int. Cl.⁴: **G 01 N 33/547**, G 01 N 33/569

(21) Anmeldenummer: **85111181.5**

(22) Anmeldetag: **04.09.85**

(54) **Trägermaterial zur Verwendung für Immunbestimmungen.**

(30) Priorität: **28.09.84 DE 3435744**

(43) Veröffentlichungstag der Anmeldung:
**02.04.86 Patentblatt 86/14**

(45) Bekanntmachung des Hinweises auf die Patenterteilung:
**08.11.89 Patentblatt 89/45**

(84) Benannte Vertragsstaaten:
**AT BE CH DE FR GB IT LI LU NL SE**

(56) Entgegenhaltungen:
**US-A- 4 046 723**

**EUROPEAN JOURNAL OF BIOCHEMISTRY, Band 73, 1977, J. SJÖDAHL "Repetitive Sequences in Protein A from Staphylococcus aureus 2", pp. 343-351**

(73) Patentinhaber: **ORPEGEN Medizinisch-Molekularbiologische Forschungsgesellschaft m.b.H, Czerny-Ring 22, D-6900 Heidelberg (DE)**

(72) Erfinder: **Scheefers, Hans, Dr., Ludwigstrasse 46, Gross-Linden D-6300 Giessen (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

## Beschreibung

Die Erfindung betrifft ein Trägermaterial zur Verwendung für die Immunbestimmung, bei dem ein Reaktionspartner der immunologischen Reaktion an das Trägermaterial kovalent gebunden ist sowie ein Verfahren zur Immunbestimmung.

Verfahren zur Immunbestimmung sind weitverbreitet. Häufig wird das ELISA-Verfahren angewendet, bei dem ein Reaktionspartner einer Immunreaktion an eine feste Phase adsorbiert vorliegt. Als feste Phase verwendet man in der Praxis dabei bevorzugt Kunststoffröhrchen oder Mikrotiterplatten, die an der Innenoberfläche adsorbiert den einen Partner der Reaktion tragen. Ebenso üblich ist es, Kugeln zu verwenden, die an ihrer Aussenoberfläche adsorbiert den einen Partner der Reaktion tragen. Die gängigsten drei Verfahren zur Enzymimmunbestimmung sind nun das Sandwich-Verfahren, das indirekte Verfahren und das Kompetitionsverfahren. Bei dem Sandwich-Verfahren wird ein Antikörper an den Träger adsorbiert, die Testlösung wird zugegeben, wobei das in der Testlösung enthaltene spezifische Antigen an den Antikörper gebunden wird. Dann wird ein enzymmarkierter spezifischer Antikörper für den Antigen-Antikörper-Komplex zugegeben, der dann an den Komplex bindet. Zuletzt gibt man das für das Enzym spezifische Substrat zu. Dieses Substrat reagiert in spezifischer Weise. Die Reaktion kann beispielsweise photometrisch ausgewertet werden. Durch Messung der Lichtabsorption oder der optischen Dichte kann dann die Menge an Antigen berechnet werden, die der Absorption bzw. Dichte direkt proportional ist. Beim indirekten Verfahren wird ein Antigen an dem Trägermaterial adsorbiert. Man gibt Testlösung dazu, wobei der in der Testlösung enthaltene, für das adsorbierte Antigen spezifische Antikörper mit dem Antigen reagiert. Bei Zugabe von enzymmarkiertem Antiglobulin bindet das Antiglobulin an den Antigen-/-Antikörper-Komplex. Nach Zugabe eines für das Enzym spezifischen Substrates bildet sich wieder eine Farbe, die durch photometrische Messung ausgewertet werden kann und direkt proportional zur Menge des unbekannten Antikörpers in dem Testserum ist. Bei der dritten Methode, dem Kompetitionsverfahren, wird einer der beiden Partner der Immunreaktion an das Trägermaterial adsorbiert. Man gibt dazu eine Lösung, die sowohl den unbekannten anderen Partner der Immunreaktion enthält, als auch eine bekannte Menge an enzymmarkiertem anderem Partner der Immunreaktion. Beide Partner binden nun in Konkurrenz mit dem am Träger adsorbierten Partner der Immunreaktion. Bei einer zweiten Probe gibt man eine Standardlösung dazu, die nur markierten Partner enthält. Beiden Lösungen gibt man dann das Enzymsubstrat zu und bestimmt durch photometrische Messung die Farbbildung. Die Differenz zwischen Standard und Probe läßt eine Berechnung der Menge an unbekanntem Reaktionspartner zu.

Häufig werden auch Radioimmunbestimmungen durchgeführt. Der RIA wird verwendet als Test zum Nachweis und zur quantitativen Bestimmung von Antigen oder Antikörper durch radioaktive Markierung eines der beiden Reaktionspartner. Er kann durchgeführt werden als Festphasentest, wobei einer der beiden Reaktionspartner an eine feste Phase gebunden ist, wodurch die Abtrennung der Antigen-Antikörper-Komplexe vom freien Partner wesentlich erleichtert wird. Der Test wird auf dem Prinzip der kompetitiven Hemmung durchgeführt. Dabei wird die Bindung eines radioaktiv markierten Antigens durch den spezifischen Antikörper durch ein nicht markiertes Antigen konzentrationsabhängig gehemmt. Je grösser der Anteil des unmarkierten und zu messenden Antigens, desto geringer ist die Radioaktivität der Antigen-Antikörper-Komplexe. Bei dem Festphasen-RIA ist auch der direkte Bindungstest möglich.

Bei allen diesen Verfahren kann das Trägermaterial und der daran adsorbierte Partner der Immunreaktion nur einmal für den Test verwendet werden, danach wird beides gewöhnlich weggeworfen, da der gebundene Reaktionspartner der Immunreaktion adsorptiv an das Trägermaterial gebunden ist über starke und schwache Wechselwirkungen, sekundäre, nichtkovalente lipophile-, Dipol-Dipol- oder Ionen-Dipol-Interaktionen. Alle diese Bindungen halten den drastischen Bedingungen, die bei einer Regeneration zur Dissoziation der Antigen-Antikörper-Bindung führen, nicht stand. Ein gewisser Teil des am Trägermaterial gebundenen Partners der Immunreaktion löst sich bei dem Regenerierungsprozeß ab, so daß ein mehrmaliger Gebrauch der bisher bekannten Träger nicht möglich ist.

Aus An. Ref. Biochem. 35 (I) 1966, Seite 879, ist es bereits bekannt, Proteine über bifunktionelle Reagenzien an Trägermaterialien zu binden. Auch aus DE-OS 2 523 207 ist es bekannt, Biopolymere über Träger zu binden. Es ist jedoch nicht angegeben, wie eine derartige kovalente Bindung ausgeführt werden soll. Aus Angewandte Chemie, 84. Jahrgang, 1972, H. 8. Seiten 319–330 ist es bekannt, die kovalente Bindung über funktionelle Gruppen, die an dem Trägermaterial vorliegen, durchzuführen. Dazu muß also der verwendete Träger über reaktive Gruppen verfügen, die den Reaktanten über den Brückenbildner kovalent binden können. Es war daher wünschenswert, eine Möglichkeit zu finden, so daß ein Protein oder der Partner einer immunologischen Reaktion kovalent an den Träger gebunden werden kann, ohne daß zuvor erst eine Derivatisierung durchgeführt werden müßte.

Es war deshalb sehr wünschenswert, wiederverwendbares Trägermaterial für Immunbestimmungen zu finden, bei dem ein Reaktionspartner so an das Trägermaterial gebunden ist, daß er bei der Regenerierungsreaktion nicht abgelöst wird.

Es war nun Aufgabe der Erfindung, ein Trägermaterial zu schaffen, das einen Partner einer Immunreaktion gebunden enthält und das mehrmals wiederverwendbar ist.

Diese Aufgabe wird gelöst durch ein Trägermaterial zur Verwendung für Immunbestimmungen,

bei dem ein Reaktionspartner der immunologischen Reaktion an das Trägermaterial kovalent gebunden ist, das dadurch gekennzeichnet ist, daß der Reaktionspartner über heterobifunktionelle photoaktivierbare Verbindungen, deren eine Gruppe durch eine Arylazidgruppe gebildet wird, kovalent an Polyäthylen gebunden ist. Dazu werden bevorzugt die folgenden Verbindungen als Brückenbildner verwendet:

- N-5-Azido-2-nitrobenzoyloxysuccinimid (ANB-NOS)
- p-Azidophenacyl bromid
- p-Azidophenylglyoxal
- 4-Fluor-3-nitrophenyl-azid
- Methyl-4-azidobenzoimidat-HCl
- N-Succinimidyl-(4-azidophenyldithio)-propionat
- N-(4-Azidophenylthio)phthalimid
- N-Hydroxysuccinimidyl-4-azido-benzoat
- N-Hydroxysuccinimidyl-4-azidosalicylsäure
- N-Succinimidyl-6(4'-azido-2'-nitrophenyl-amino)-hexanoat
- Sulfosuccinimidyl-6-(4'-azido-2'-nitro-phenylamino)-hexanoat
- Ethyl-4-azidophenyl-1,4-dithiobutyrimidat-HCl
- N-Succinimidyl-(4-azidophenyldithio)-propionat
- Sulfosuccinimidyl-(4-azidophenyldithio)-propionat
- 4,4'-dithio-bis-phenylazide.

Besonders bevorzugt wird Lomant's Reagenz II: N-Succinimidyl-6-(4'-azido-2'-nitrophenyl-amino)-hexanoat verwendet.

Ein so gebundener Reaktionspartner löst sich auch unter drastischen Bedingungen nicht von dem Trägermaterial, so daß ein derartig behandeltes Polyäthylen sehr oft wiederverwendbar ist.

Das Trägermaterial wird üblicherweise in Form von Kugeln, Mikrotiterplatten oder Röhrchen verwendet. Dieses Trägermaterial besteht aus Polyäthylen das, die Eigenschaft besitzt, möglichst wenig Reaktionspartner adsorptiv zu binden.

Als besonders geeignet hat sich dabei ein oberflächenbehandeltes Polyethylen erwiesen, wie es beispielsweise unter der Bezeichnung «Minisorpmaterial» in Form von Röhrchen von der Firma NUNC, Dänemark, vertrieben wird.

Das erfindungsgemäße Trägermaterial eignet sich für alle Arten von Immunbestimmungen, insbesondere für ELISA und Festphasen-RIA).

An das Trägermaterial gebunden werden können alle Reaktionspartner einer immunologischen Reaktion. So kann z. B. ein Antigen, ein Allergen oder ein Antikörper kovalent an den Träger fixiert werden.

Der Reaktionspartner wird über heterobifunktionelle photoaktivierbare Verbindungen, deren eine bifunktionelle Gruppe durch eine Arylazidgruppe gebildet wird, an das Trägermaterial gebunden. Bevorzugt werden als Brückenbildner die im Anspruch 2 aufgeführten Verbindungen verwendet. Besonders bevorzugt ist die als Lohmant's Reagenz II bekannte Verbindung N-Succinimidyl-6-(4'-azido-2'-nitrophenylamino)-Hexan, die auf der einen Seite eine Arylacidgruppe trägt, die sich in einer Photolysereaktion, ausgelöst durch sichtbares Licht, unter Bildung eines Nitrens umsetzen läßt. Das bei der Photolyse gebildete Nitren ist äußerst reaktiv. Es geht unselektiv bezüglich seines Reaktionspartners eine kovalente Bindung mit seinem nächsten Nachbarn ein. Dieses, durch Lichteinwirkung generierte Nitren, kann auch mit relativ inertem Material reagieren und ist bei der Reaktion nicht auf Reaktivgruppen eines bestimmten, aus besonderen Gründen anzuwendenden Materials angewiesen. Es reagiert daher mit dem chemisch relativ inerten Polyethylen. Die andere funktionelle Gruppe des bevorzugten Brückenbildners ist ein N-Hydroxy-Succinimidester, der bei alkalischem pH sehr schonend mit nicht protonierten freien Aminogruppen eines Reaktanten, wie z. B. einem Protein, reagiert. Auf diese Weise ist es möglich, z. B. Antigene, Allergene und Antikörper zu binden.

Eine weitere Ausführungsform der Erfindung besteht darin, über den Brückenbildner zunächst Protein A (Agglutinogen aus Staphylococcus aureus mit vier homologen Bindungsstellen für die Fc-Region von Immunoglobulin G: Sjödahl, J. (1977) Eur. J. Biochem. 73 343–51) kovalent an das Trägermaterial zu binden. Zugefügte Antikörper können dann mit ihrem konstanten Teil an das kovalent mit Protein A beschichtete Trägermaterial binden und die variablen Teile der Antikörper sind dann für die Bindung des anderen Partners der immunologischen Reaktion verfügbar. Eine so gerichtete Beladung des Trägers erhöht die biologische Effizienz der Beschichtung außerordentlich und macht das System, bezogen auf die verwendbare Trägerfläche, empfindlicher. Es besteht dann auch die Möglichkeit, den kovalent mit dem Protein A beschichteten Träger für eine weitere chemische Reaktion zu verwenden. Bei dieser chemischen Reaktion kann man unter Verwendung eines Proteinbrückenbildners den zuvor gerichtet adsorptiv an das Protein A gebundenen Antikörper kovalent mit diesem vernetzen, so daß nach der Vernetzungsreaktion der Antikörper gerichtet und kovalent gebunden am Träger vorliegt.

Der über den Brückenbildner gebundene Reaktionspartner hat einen gewissen Abstand von dem Trägermaterial und auch eine gewisse Mobilität. Dies wirkt sich sehr günstig auf die Zugänglichkeit des kovalent gebundenen Reaktionspartners aus, was auch wiederum zu einer Verbesserung der Immunreaktion führt.

Das erfindungsgemäße Trägermaterial, das den einen Partner einer Immunreaktion kovalent gebunden über einen Brückenbildner trägt, kann häufig wiederverwendet werden, da sich der Reaktionspartner auch unter den drastischen Bedingungen, die für die Regenerierung erforderlich sind, nicht von dem Trägermaterial ablöst, da er ja kovalent und nicht adsorptiv gebunden vorliegt.

Das erfindungsgemäße Verfahren für die Immunbestimmung, bei dem einer der an der Immunreaktion beteiligten Partner kovalent an Polyäthylen als Trägermaterial gebunden wird, ist dadurch gekennzeichnet, daß die Bindung über eine heterobifunktionelle, photoaktivierbare Verbindung, deren eine Gruppe durch eine Arylacidgruppe gebildet wird, vorzugsweise über N-Succinimidyl-6(4'-azido-2'-nitrophenyl-amino)-Hexan, als Brückenbildner erfolgt.

Das Trägermaterial mit dem daran gebundenen Partner kann nach der Immunreaktion wiedergewonnen und erneut für die Immunbestimmung verwendet werden.

Dieses Verfahren kann mit dem erfindungsgemäßen Trägermaterial beliebig oft wiederholt werden, da sich der kovalent gebundene Partner auch unter den drastischen Reaktionsbedingungen der Regenerierung nicht ablöst und gebunden bleibt.

In einer weiteren Ausführungsform des erfindungsgemäßen Verfahrens wird an den Träger Protein A kovalent gebunden, das dann einen Antikörper trägt. Auf diese Weise werden die konstanten Teile des Antikörpers gebunden und die variablen Teile des Antikörpers stehen für die imunologische Reaktion zur Verfügung. Nach Durchführung der immunologischen Reaktion kann dann der Träger mit dem kovalent gebundenen Protein A und dem daran adsorptiv oder kovalent gebundenen Antikörper wiedergewonnen werden durch Regenerierung.

Das erfindungsgemäße Trägermaterial und das Verfahren zur Immunbestimmung haben mehrere Vorteile. Ein und dasselbe Trägermaterial läßt sich sehr oft nach jeweils geeigneten Regenerierungsschritten für Immunbestimmungen verwenden. Besonders vorteilhaft ist das erfindungsgemäße wiederverwendbare Trägermaterial für Immunbestimmungen, in denen als zu bindender Reaktionspartner ein Partner verwendet wird, der nur in begrenzter Menge vorhanden ist, da er sich schwierig herstellen läßt oder schierig zu isolieren oder synthetisieren ist. In diesen Fällen läßt sich die zur Verfügung stehende Menge an Reaktionspartner an derivatisiertes Trägermaterial koppeln und es ist auf diese Weise möglich, wesentlich mehr Immunobestimmungen mit einer begrenzt vorliegenden Menge an Reaktionspartner auszuführen. Weiterhin werden die Kosten für die einzelne Bestimmung erheblich gesenkt.

Beispiel

Es wurde Protein A mit der als Brückenbildner verwendeten Verbindung N-Succinimidyl-6-(4'-azido-2'-nitrophenyl-amino)-Hexan an Polyäthylen gebunden. Nach entsprechenden Waschschritten wurde das trägergebundene Protein A ohne Lichteinwirkung mit dem Lohmant's Reagenz bei alkalischem pH-Wert reagieren gelassen. Dieser zweite Brückenbildner reagierte unter diesen Bedingungen mit dem N-Hydroxy-Succinimidester als funktionelle Gruppe kovalent mit dem Träger gebundenen Protein A. Nach weiteren Waschschritten wurde das derivatisierte trägergebundene Protein A mit Antikörpern inkubiert, welche hierbei mit ihrem konstanten Teil an das Protein A gebunden wurden. Nach erneuten weiteren Waschschritten konnte man durch eine photochemische Reaktion Antikörper, gerichtet an das Protein A, kovalent binden, wobei der variable Teil des Antikörpers jeweils der Trägeroberfläche abgewandt und dem zu bindenden Antigen zugewandt war. Der so derivatisierte Träger hatte eine höhere Beladungsdichte und eine daraus resultierende höhere enzymatische Aktivität pro Fläche. Er konnte für 100 Bestimmungen verwendet werden.

Patentansprüche

1. Trägermaterial zur Verwendung für die Immunbestimmung, bei dem ein Reaktionspartner der immunologischen Reaktion an das Trägermaterial kovalent gebunden ist, dadurch gekennzeichnet, daß der Reaktionspartner über hererobifunktionelle photoaktivierbare Verbindungen, deren eine Gruppe durch eine Arylazidgruppe gebildet wird, kovalent an Polyäthylen gebunden ist.

2. Trägermaterial nach Anspruch 1 dadurch gekennzeichnet, daß als heterobifunktionelle photoaktivierbare Verbindung
- N-5-Azido-2-nitrobenzoyloxysuccinimid (ANB-NOS)
- p-Azidophenacyl bromid
- p-Adizophenylglyoxal
- 4-Fluoro-3-nitrophenyl azid
- Methyl-4-azidobenzoimidat-HCl
- NSuccinimidyl-(4-azidophenyldithio)-propionat
- N-(4-azidophenylthio)phthalimid
- N-Hydroxysuccinimidyl-4-azido-benzoat
- N-Hydroxysuccinimidyl-4-azidosalicylsäure
- Sulfosuccinimidyl-6-(4'-azido-2'-nitro-phenylamino)-hexanoat
- Ethyl-4-azidophenyl-1,4-dithiobutyrimidat-HCl
- N-Succinimidyl-(4-azidophenyldithio)-propionat
- Sulfosuccinimidyl-(4-azidophenyldithio)-propionat und/oder
- 4,4'-Dithio-bis-phenylazide verwendet werden.

3. Trägermaterial nach Anspruch 1, dadurch gekennzeichnet, daß die heterobifunktionelle photoaktivierbare Verbindung N-Succinimidyl-6-(4'-azido-2'-nitrophenyl-amino)-Hexanoat ist.

4. Trägermaterial nach Anspruch 1, dadurch gekennzeichnet, daß an den Brückenbildner ein Protein gebunden ist, das einen Reaktionspartner der immunologischen Reaktion trägt.

5. Verfahren zur Herstellung eines Trägermaterials nach einem der Ansprüche 1 bis 3, dadurch gekennzeichnet, daß man einen Reaktionspartner der immunologischen Reaktion über eine heterobifunktionelle photoaktivierbare Verbindung, deren eine Gruppe durch eine Arylazidgruppe gebildet wird, kovalent an Polyäthylen bindet.

6. Verfahren zur Herstellung eines Trägermaterials nach Anspruch 4, dadurch gekennzeichnet, daß man Protein A über eine heterobifunktionelle photoaktivierbare Verbindung, deren eine Gruppe durch eine Arylazidgruppe gebildet wird, kovalent an Polyäthylen bindet, dann als Reaktionspartner der Immunreaktion einen Antikörper mit seinem konstanten Teil an das Protein A bindet unter Ausbildung einer gerichteten Bindung des Antikörpers an den Träger.

7. Verfahren nach Anspruch 6, dadurch gekennzeichnet, daß man den an das Protein A gebundenen Antikörper kovalent mit diesem vernetzt unter Verwendung heterobifunktioneller Brückenbildner.

8. Verfahren nach Anspruch 7, dadurch gekennzeichnet, daß der Brückenbildner N-Hydroxysuccinimidyl-4-azidosallicylsäure oder N-Succinimidyl-6-(4-azido-2-nitrophenyl-amino)-hexan ist.

9. Verfahren zur Immunbestimmung, bei dem einer der an der Immunreaktion beteiligten Partner kovalent an ein Trägermaterial gebunden wird, dadurch gekennzeichnet, daß man ein Trägermaterial nach einem der Ansprüche 1 bis 4 verwendet.

10. Verfahren zur Immunbestimmung dadurch gekennzeichnet, daß man nach einer Immunbestimmung gemäß Anspruch 9 das Trägermaterial mit dem daran gebundenen Partner wiedergewinnt und erneut zur Immunbestimmung verwendet.

**Patentansprüche für: AT**

1. Verfahren zur Herstellung eines Trägermaterials zur Verwendung für die Immunbestimmung, bei dem ein Reaktionspartner der immunologischen Reaktion an das Trägermaterial kovalent gebunden ist, dadurch gekennzeichnet, daß man einen Reaktionspartner der immunologischen Reaktion über eine heterobifunktionelle photoaktivierbare Verbindung, deren eine Gruppe durch eine Arylazidgruppe gebildet wird, kovalent an Polyäthylen bindet.

2. Verfahren zur Herstellung eines Trägermaterials nach Anspruch 1, dadurch gekennzeichnet, daß man Protein A über eine heterobifunktionelle photoaktivierbare Verbindung, deren eine Gruppe durch eine Arylazidgruppe gebildet wird, kovalent an Polyäthylen bindet, dann als Reaktionspartner der Immunreaktion einen Antikörper mit seinem konstanten Teil an das Protein A bindet unter Ausbildung einer gerichteten Bindung des Antikörpers an den Träger.

3. Verfahren nach Anspruch 1 oder 2, dadurch gekennzeichnet, daß man als heterobifunktionelle photoaktivierbare Verbindung
– N-5-Azido-2-nitrobenzoyloxysuccinimid (ANB-NOS)
– p-Azidophenacylbromid
– p-Azidophenylglyoxal
– 4-Fluoro-3-nitrophenylazid
– Methyl-4-azidobenzoimidat-HCl
– N-Succinimidyl-(4-azidophenyldithio)-propionat
– N-(4-azidophenylthio)-phthalimid
– N-Hydroxysuccinimidyl-4-azido-benzoat
– N-Hydroxysuccinimidyl-4-azidosalicylsäure
– Sulfosuccinimidyl-6-(4'-azido-2'-nitrophenyl-amino)-hexanoat
– Ethyl-4-azidophenyl-1,4-dithiobutyrimidat-HCl
– N-Succinimidyl-(4-azidophenyldithio)-propionat
– Sulfosuccinimidyl-(4-azidophenyldithio)-propionat und oder
– 4,4'-Dithio-bis-phenylazide verwendet.

4. Verfahren nach Anspruch 3, dadurch gekennzeichnet, daß man die heterobifunktionelle photoaktivierbare Verbindung N-Succinimidyl-6-(4'-azido-2'-nitrophenyl-amino)-Hexanoat verwendet.

5. Verfahren nach einem der Ansprüche 1 bis 4, dadurch gekennzeichnet, daß an den Brückenbildner ein Protein gebunden ist, das einen Reaktionspartner der immunologischen Reaktion trägt.

6. Verfahren nach Anspruch 5, dadurch gekennzeichnet, daß man einen an das Protein A gebundenen Antikörper kovalent mit diesem vernetzt unter Verwendung heterobifunktioneller Brückenbildner.

7. Verfahren nach Anspruch 6, dadurch gekennzeichnet, daß der Brückenbildner N-Hydroxysuccinimidyl-4-azidosallicylsäure oder N-Succinimidyl-6-(4-azido-2-nitrophenyl-amino)-hexan ist.

8. Verfahren zur Immunbestimmung, bei dem einer der an der Immunreaktion beteiligten Partner kovalent an ein Trägermaterial gebunden wird, dadurch gekennzeichnet, daß man ein Trägermaterial, das nach einem der Ansprüche 1 bis 7 erhalten ist, verwendet.

9. Verfahren zur Immunbestimmung, dadurch gekennzeichnet, daß man nach einer Immunbestimmung gemäß Anspruch 8 das Trägermaterial mit dem daran gebundenen Partner wiedergewinnt und erneut zur Immunbestimmung verwendet.

**Claims for the Contracting States: BE CH DE FR GB IT LI LU NL SE**

1. Support material for use for immune determinations in which one reaction component of the immunological reaction is covalently bound to the support material, characterised in that the reaction component is covalently bound to polyethylene via heterobifunctional photoactivatable compounds, one group of which is formed by an aryl azide group.

2. Carrier material according to claim 1, characterised in that as heterobifunctional photoactivatable compound are used
N-5-azido-2-nitrobenzoyloxysuccinimide (ANB-NOS),
p-azidophenacyl bromide,
p-azidophenyl-glyoxal,
4-fluoro-3-nitrophenyl azide,
methyl 4-azido-benzoimidate HCl,
N-succinimidyl (4-azidophenyldithio)-propionate,
N-(4-azidophenylthio)-phthalimide,
N-hydroxysuccinimidyl-4-azidobenzoate,
N-hydroxysuccinimidyl-4-azidosalicylic acid,
sulphosuccinimidyl 6-(4'-azido-2'-nitrophenyl amino)-hexanoate,
ethyl 4-azidophenyl-1,4-dithiobutyrimidate HCl,
N-succinimidyl (4-azidophenyldithio)-propionate,
sulphosuccinimidyl (4-azidophenyldithio)-propionate and/or
4,4'-dithio-bis-phenylazide.

3. Carrier material according to claim 1, caracterised in that the heterobifunctional photoactivatable compound is N-succinimidyl 6-(4'-azido-2'-nitrophenyl-amino)-hexanoate.

4. Carrier material according to claim 1, characterised in that a protein which carries a reaction component of the immunological reaction is bound to the bridge builder.

5. Process for the preparation of a support material according to one of claims 1 to 3, characterised in that one covalently binds to polyethylene a reaction component of the immunological reaction via a heterobifunctional photoactivatable compound, one group of which is formed by an aryl azide group.

6. Process for the preparation of a support material according to claim 4, characterised in that one covalently binds protein A to polyethylene via a heterobifunctional photoactivatable compound, one group of which is formed by an aryl azide group, then as reaction component of an immune reaction, binds an antibody with its constant part to the protein A with formation of a directed binding of the antibody to the support.

7. Process according to claim 6, characterised in that one cross-links the antibody bound to the protein A covalently with this with the use of heterobifunctional bridge builders.

8. Process according to claim 7, characterised in that the bridge builder is N-hydroxysuccinimidyl-4-azidosalicylic acid or N-succinimidyl-6-(4'-azido-2'-nitrophenylamino)-hexane.

9. Process for immune determination, in which one of the components participating in the immune reaction is covalently bound to a support material, characterised in that one uses a carrier material according to one of claims 1 to 4.

10. Process for immune determination, characterised in that one recovers the support material with the component bound thereon after an immune determination according to claim 9 and uses again for the immune determination.

**Claims for the Contracting State: AT**

1. Process for the preparation of a support material for use for the immune determination in which one of the reaction components of the immunological reaction is covalently bound to the support material, characterised in that one covalently binds to polyethylene a reaction component of the immunological reaction via a heterobifunctional photoactivatable compound, one group of which is formed by an aryl azide group.

2. Process for the preparation of a support material according to claim 1, characterised in that one covalently binds protein A to polyethylene via a heterobifunctional photoactivatable compound, one group of which is formed by an aryl azide group, then, as reaction component of the immune reaction, binds an antibody with its constant part to the protein A with formation of a directed binding of the antibody to the support.

3. Process according to claim 1 or 2, characterised in that, as heterobifunctional photoactivatable compound, one uses

N-5-azido-2-nitrobenzoyloxysuccinimide (ANB-NOS),

p-azidophenacyl bromide,
p-azidophenylglyoxal,
4-fluoro-3-nitrophenyl azide,
methyl 4-azidobenzoimidate HCl,
N-succinimidyl (4-azidophenyldithio)-propionate,
N-(4-azidophenylthio)-phthalimide,
N-hydroxysuccinimidyl 4-azidobenzoate,
N-hydroxysuccinimidyl 4-azidosalicylic acid,
sulphosuccinimidyl 6-(4'-azido-2'-nitrophenyl-amino)-hexanoate,
ethyl 4-azidophenyl-1,4-dithio-butyrimidate HCl,
N-succinimidyl (4-azidophenyldithio)-propionate,
sulphosuccinimidyl (4-azidophenyldithio)-propionate and/or
4,4'-dithio-bis-phenylazide.

4. Process according to claim 3, characterised in that one uses the heterobifunctional photoactivatable compound N-succinimidyl 6-(4'-azido-2'-nitrophenyl-amino)-hexanoate.

5. Process according to one of claims 1 to 4, characterised in that to the bridge builder is bound a protein which carries a reaction component of the immunological reaction.

6. Process according to claim 5, characterised in that one cross-links the antibody bound to the protein A covalently with this with the use of heterobifunctional bridge builders.

7. Process according to claim 6, characterised in that the bridge builder is N-hydroxysuccinimidyl-4-azidosalicylic acid or N-succinimidyl-6-(4'-azido-2'-nitrophenylamino)-hexane.

8. Process for immune determination in which one of the components participating in the immune reaction is covalently bound to a support material, characterised in that a carrier material is used which is obtained according to one of claims 1 to 7.

9. Process for immune determination, characterised in that one recovers the support material with the component bound thereon after an immune determination according to claim 8 and uses again for the immune determination.

**Revendications pour les Etats contractants: BE CH DE FR GB IT LI LU NL SE**

1. Matière de support pour l'utilisation pour le dosage immunologique, dans laquelle un partenaire réactionnel de l'immunoréaction est lié à la matière de support par covalence, caractérisée en ce que le partenaire réactionnel est lié par covalence à du polyéthylène par l'intermédiaire de composés photoactivables hétérobifonctionnels, dont un groupe est formé par un groupe arylazide.

2. Matière de support suivant la revendication 1, carctérisée en ce qu'on utilise comme composés hétérobifonctionnels photoactivables
– le N-5-azido-2-nitrobenzoyloxysuccinimide (ANB-NOS)
– le bromure de p-azidophénacyle
– le p-azidophénylglyoxal

– le 4-fluoro-3-nitrophényl azide
– le méthyl-4-azidobenzoimidate-HCl
– le N-succinimidyl-(4-azidophényldithio)-propionate
– le N-(4-azidophénylthio)phtalimide
– le N-hydroxysuccinimidyl-4-azido-benzoate
– l'acide N-hydroxysuccinimidyl-4-azido-salicylique
– le sulfosuccinimidyl-6-(4'-azido-2'-nitro-phénylamino) hexanoate
– l'éthyl-4-azidophényl-1,4-dithiobutyrimidate-HCl
– le N-succinimidyl-(4-azidophényldithio-propionate
– le sulfosuccinimidyl-(4-azidophényldithio-propionate et/ou
– le 4,4'-dithio-bis-phénylazide.

3. Matière de support suivant la revendication 1, caractérisée en ce que le composé hétérobifonctionnel photoactivable est le N-succinimidyl-6-(4'-azido-2'-nitrophényl-amino)-hexanoate.

4. Matière de support suivant le revendication 1, caractérisée en ce qu'une protéine est liée sur l'agent de pontage, laquelle porte un partenaire réactionnel de l'immunoréaction.

5. Procédé de préparation d'une matière de support suivant l'une des revendications 1 à 3, caractérisé en ce qu'on lie par covalence à du polyéthylène un partenaire réactionnel de l'immunoréaction par l'intermédiaire d'un composé hétérobifonctionnel photoactivable dont un groupe est formé par un groupe arylazide.

6. Procédé de préparation d'une matière de support suivant la revendication 4, caractérisé en ce qu'on lie par covalence à du polyéthylène une protéine A par l'intermédiaire d'un composé hétérobifonctionnel photoactivable dont un groupe est formé par un groupe arylazide, puis en ce qu'on lie, comme partenaire réactionnel de l'immunoréaction, un anticorps avec sa partie constante à la protéine A avec formation d'une liaison dirigée de l'anticorps sur le support.

7. Procédé suivant la revendication 6, caractérisé en ce qu'on réticule l'anticorps lié à la protéine A par covalence avec celle-ci en utilisant un agent de pontage hétérobifonctionnel.

8. Procédé suivant la revendication 7, caractérisé en ce que l'agent de pontage est l'acide N-hydroxysuccinimidyl-4-azidosalicylique ou le N-succinimidyl-6-(4-azido-2-nitrophényl-amino)-hexane.

9. Procédé de dosage immunologique dans lequel un des partenaires participant à l'immunoréaction est lié par covalence à une matière de support caractérisée en ce qu'on utilise une matière de support suivant l'une des revendications 1 à 4.

10. Procédé de dosage immunologique, caractérisé en ce qu'on récupère la matière de support après un dosage immunologique suivant la revendication 9 avec le partenaire lié à celle-ci, et en ce qu'on l'utilise à nouveau pour le dosage immunologique.

**Revendications pour l'Etat contractant: AT**

1. Procédé de préparation d'une matière de support pour l'utilisation pour le dosage immunologique, dans lequel un partenaire réactionnel de l'immunoréaction est lié par covalence à la matière de support, caractérisé en ce qu'on lie par covalence à du polyéthylène un partenaire réactionnel de l'immunoréaction par l'intermédiaire d'un composé hétérobifonctionnel photoactivable, dont un groupe est formé par un groupe arylazide.

2. Procédé de préparation d'une matière de support suivant las revendication 1, caractérisé en ce qu'on lie par covalence à du polyéthylène une protéine A par l'intermédiaire d'un composé hétérobifonctionnel photoactivable dont un groupe est formé par un groupe arylazide, puis en ce qu'on lie comme partenaire réactionnel de l'immunoréaction un anticorps avec sa partie constante à la protéine A, avec formation d'une liaison dirigée de l'anticorps sur le support.

3. Procédé suivant les revendications 1 ou 2, caractérisé en ce qu'on utilise comme composé hétérobifonctionnel photoactivable
– le N-5-azido-2-nitrobenzoyloxysuccinimide (ANB-NOS)
– le bromure de p-azidophénacyle
– le p-azidophénylglyoxal
– le 4-fluoro-3-nitrophénylazide
– le méthyl-4-azidobenzoimidate-HCl
– le N-succinimidyl-(4-azidophényldithio)-propionate
– le N-(4-azidophénylthio)-phtalimide
– le N-hydroxysuccinimidyl-4-azido-benzoate
– l'acide N-hydroxysuccinimidyl-4-azido-salicylique
– le sulfosuccinimidyl-6-(4'-azido-2'-nitro-phényl-amino)-hexanoate
– l'éthyl-4-azidophényl-1,4-dithiobutyrimidate-HCl
– le N-succinimidyl-(4-azidophényldithio)-propionate
– le sulfosuccinimidyl-(4-azidophényldithio)-propionate er/ou
– le 4,4'-dithio-bis-phénylazide.

4. Procédé suivant la revendication 3, caractérisé en ce qu'on utilise comme composé hétérobifonctionnel photoactivable le N-succinimidyl-6-(4'-azido-2'-nitrophényl-amino)-hexanoate.

5. Procédé suivant l'une des revendications 1 à 4, caractérisé en ce qu'une protéine est liée sur l'agent de pontage, laquelle porte un partenaire réactionnel de l'immunoréaction.

6. Procédé suivant la revendication 5, caractérisé en ce qu'on réticule par covalence un anticorps lié à la protéine A avec celle-ci en utilisant un agent de pontage hétérobifonctionnel.

7. Procédé suivant la revendication 6, caractérisé en ce que l'agent de pontage est l'acide N-hydroxysuccinimidyl-4-azidosalicylique ou le N-succinimidyl-6-(4-azido-2-nitrophényl-amino)-hexane.

8. Procédé de dosage immunologique, dans lequel un des partenaires participant à l'immunoréaction est lié par covalence à une matière de support, caractérisé en ce qu'on utilise une matière de support qui a été obtenue suivant l'une des revendications 1 à 7.

9. Procédé de dosage immunologique, caractérisé en ce qu'on récupère la matière de support avec le partenaire qui lui est lié après un dosage immunologique suivant la revendication 8, et en ce qu'on l'utilise à nouveau pour le dosage immunologique.